# EUROPÄISCHE PATENTSCHRIFT

(11) **EP 4 157 213 B1**
(45) Veröffentlichungstag und Bekanntmachung des Hinweises auf die Patenterteilung: **18.03.2026**
(21) Anmeldenummer: 21722854.3
(22) Anmeldetag: 30.04.2021
(51) Int. Cl.: A61K 8/92, A61Q 17/04

(54) **SONNENSCHUTZMITTEL MIT EINER KOMBINATION AUS CARNAUBAWACHS UND HYDRIERTEM RAPSÖL**
SUNSCREEN WITH A COMBINATION OF CARNAUBA WAX AND HYDROGENATED RAPESEED OIL
ÉCRAN SOLAIRE COMPRENANT UNE COMBINAISON DE CIRE DE CARNAUBA ET D'HUILE DE COLZA HYDROGÉNÉE

(30) Priorität: 26.05.2020 DE 102020206521
(43) Veröffentlichungstag der Anmeldung: 05.04.2023
(73) Patentinhaber: Beiersdorf AG, 22529 Hamburg (DE)
(72) Erfinder: GRONAU-HORN, Annette, 22549 Hamburg (DE); FÄNGER, Sabine, 22763 Hamburg (DE); KÜSTERS, Julia, 21259 Otter (DE); JAPP, Christina, 21149 Hamburg (DE); PEIRANO, Reto, 22761 Hamburg (DE)
(74) Vertreter: Beiersdorf AG
(86) Internationale Anmeldenummer: PCT/EP2021/061405
(87) Internationale Veröffentlichungsnummer: WO 2021/239385

(56) Entgegenhaltungen:
- DE-A1- 102017 202 838
- DE-A1- 102018 203 498
- DATABASE GNPD [online] MINTEL; 31 August 2018 (2018-08-31), ANONYMOUS: "Anti-Pollution Lip Protector SPF 20", XP055830953, retrieved from https://www.gnpd.com/sinatra/recordpage/5906615/ Database accession no. 5906615

## Beschreibung

Die vorliegende Erfindung betrifft eine kosmetische Zubereitung enthaltend
a) ein oder mehrere UV-Filter,
b) Carnaubawachs (INCI Copernicia Cerifera Cera) und
c) hydriertes Rapsöl (INCI: hydrogenated rapeseed oil) sowie Verfahren und Verwendungen mit Carnaubawachs (INCI Copernicia Cerifera Cera) und hydriertem Rapsöl (INCI: hydrogenated rapeseed oil) in kosmetischen Sonnenschutzmitteln.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine weit verbreitete Nutzungsform kosmetischer Sonnenschutzmittel findet rund um den Wassersport statt. Gerne werden die Sonnenschutzmittel in Verbindung mit Strandurlaub, Besuchen in Schwimmbädern, beim Baden, Surfen, Tauchen, Paddeln oder anderen Aktivitäten eingesetzt, bei denen die menschliche Haut mit Wasser in Kontakt kommt.

Bei allen diesen Aktivitäten kommt es für einen umfassenden UV-Schutz darauf an, dass das Sonnenschutzmittel eine gewisse Wasserfestigkeit aufweist und nicht sofort bei Kontakt mit dem Wasser von der Haut abgespült wird.

Zur Erhöhung der Wasserfestigkeit kosmetischer Sonnenschutzmittel werden diesen Zubereitungen in der Regel sogenannte Filmbildner zugesetzt. Bei den Filmbildnern handelt es sich üblicherweise um polymere Verbindungen auf der Basis von Polyacrylaten oder Polyvinylpyrrolidonen, die auf der Haut einen Schutzfilm ausbilden, der das Abwaschen der UV-Filter von der Haut verhindern/verzögern soll.

Der Einsatz derartiger Filmbildner wird von einer breiten Öffentlichkeit zunehmend kritisch gesehen. Es wird in der öffentlichen Diskussion des Öfteren die Sorge geäußert, polymere Filmbildner können analog dem so genannten "Mikroplastik" eine Belastung für die Umwelt darstellen. Ob derartige Sorgen berechtigt und naturwissenschaftlich begründet sind, kann im Rahmen der vorliegenden Offenbarung dahingestellt bleiben. Tatsache ist hingegen, dass bei den Kosmetikherstellern und Verbrauchern zunehmend ein Interesse daran besteht, alternative Techniken zur Gewährleistung der Wasserfestigkeit von Sonnenschutzmitteln zu entwickeln.

Es war daher die Aufgabe der vorliegenden Erfindung, ein möglichst wasserfestes Sonnenschutzmittel zu entwickeln, bei dem auf den Einsatz von polymeren Filmbildnern (insbesondere solchen auf Basis von Polyacrylaten und Polyvinylpyrrolidonen) weitgehend verzichtet werden kann.

Überraschend gelöst wird die Aufgabe durch ein kosmetisches Sonnenschutzmittel enthaltend
a) ein oder mehrere UV-Filter,
b) Carnaubawachs (INCI Copernicia Cerifera Cera)
c) hydriertes Rapsöl (INCI: hydrogenated rapeseed oil), dadurch gekennzeichnet, dass die Zubereitung bzw. das Sonnenschutzmittel zusätzlich Cetylpalmitat (INCI: Cetyl Palmitate)
und/oder C18-38 Alkylhydroxystearoylstearat (INCI: C18-38 Alkyl Hydroxystearoyl Stearate) enthält, sowie durch ein kosmetisches Sonnenschutzmittel enthaltend
a) ein oder mehrere UV-Filter,
b) Carnaubawachs (INCI Copernicia Cerifera Cera)
c) hydriertes Rapsöl (INCI: hydrogenated rapeseed oil), dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Der bei Kontakt mit Wasser ermittelte Lichtschutzfaktor, auch SPW genannt, wird nach der folgenden Methode bestimmt:
In Vivo Determination of Sun Protection International Standard ISO 24444; 2010, Sun Product Very Water Resistance -Spa Pool, COLIPA 2005.

Erfindungsgemäß ist ferner die Verwendung einer Kombination aus Carnaubawachs (INCI Copernicia Cerifera Cera) und hydriertem Rapsöl (INCI: hydrogenated rapeseed oil) zur Erhöhung der Wasserfestigkeit kosmetischer Sonnenschutzmittel.

Die Formulierungen "erfindungsgemäß", "erfindungsgemäß vorteilhaft" etc. beziehen sich im Rahmen der vorliegenden Offenbarung immer auf die erfindungsgemäße kosmetische Zubereitung (Sonnenschutzmittel) und die erfindungsgemäßen Verwendungen, wenn es im Einzelfall nicht anders beschrieben wird.

Zwar kennt der Stand der Technik die DE102018203498 A1, DE102017202838 A1 sowie den Eintrag in der GNPD-Datenbank Mintel "Anti-Pollution Lip Protector SPF 20" mit der Eintragungsnummer 5906615, doch konnten diese Publikationen nicht den Weg zur vorliegenden Erfindung weisen.

Als UV-Filter gelten erfindungsgemäß alle Substanzen, die in der Verordnung (EU) Nr. 866/2014 und der Verordnung Nr. 1223/2009 der EU Kommission in der Anlage VI als UV-Filter aufgelistet sind.

Es ist erfindungsgemäß bevorzugt, wenn die das Sonnenschutzmittel frei ist von Polyacrylaten, Carbomeren und Polyvinylpyrrolidonen.

Erfindungsgemäß vorteilhafte Ausführungsformen sind dadurch gekennzeichnet, dass das Sonnenschutzmittel zusätzlich Cetylpalmitat (INCI: Cetyl Palmitate) und/oder C18-38 Alkylhydroxystearoylstearat (INCI: C18-38 Alkyl Hydroxystearoyl Stearate) enthält.

Erfindungsgemäß besonders bevorzugt ist es dabei, wenn die Zubereitung bzw. das Sonnenschutzmittel Cetylpalmitat (INCI: Cetyl Palmitate) und C18-38 Alkylhydroxystearoylstearat (INCI: C18-38 Alkyl Hydroxystearoyl Stearate) enthält.

Erfindungsgemäß vorteilhafte Ausführungsformen der vorliegenden Erfindung sind dadurch gekennzeichnet, dass die Zubereitung einen oder mehrere UV-Filter gewählt aus der Gruppe der

Verbindungen 4-(tert.-Butyl)-4'-methoxydibenzoylmethan (INCI Butyl Methoxydibenzoyl-methane), (2-[-4-(Diethylamino)-2-hydroxybenzoyl] Benzoesäurehexylester (INCI: Diethyl-amino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

Enthält die Zubereitung 4-(tert.-Butyl)-4'-methoxydibenzoylmethan (INCI Butyl Methoxydibenzoylmethane), so beträgt die erfindungsgemäß vorteilhafte 1 bis 5% Gew.-%, bezogen auf das Gesamtgewicht der Zubereitung.

Enthält die Zubereitung (2-[-4-(Diethylamino)-2-hydroxybenzoyl] Benzoesäurehexylester (INCI: Diethylamino Hydroxybenzoyl Hexyl Benzoate), so beträgt die erfindungsgemäß vorteilhafte Einsatzkonzentration von 1 bis 9 Gew.-%, bezogen auf das Gesamtgewicht der Zubereitung.

Enthält die Zubereitung 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), so beträgt die erfindungsgemäß vorteilhafte Einsatzkonzentration von 0,5 bis Gew. 4,5% bezogen auf das Gesamtgewicht der Zubereitung.

Ferner sind erfindungsgemäß vorteilhafte Ausführungsformen dadurch gekennzeichnet, dass sie einen oder mehrere UV-Filter, gewählt aus der Gruppe der Verbindungen 2-Phenylbenzimidazol-5-sulfonsäuresalze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäuresalze; 2-Ethylhexyl-2-cyano-3,3-diphenylacrylat; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysilo-xan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-ben-zoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid, enthalten.

Dabei ist insbesondere der Einsatz von Phenylbenzimidazol-5-sulfonsäuresalzen, Ethylhexylsalicylat, Homomenthylsalicylat, 2-Ethylhexyl-2-cyano-3,3-diphenylacrylat, Dioctylbutylamidotriazon (INCI: Diethylhexyl-Butamidotriazone), 4,4',4"-(1,3,5-Triazin-2,4,6-triyltriimino)-tris-benzoësäure-tris(2-ethylhexylester) (INCI: Ethylhexyl Triazone), Titandioxid. erfindungsgemäß bevorzugt. Erfindungsgemäß besonders bevorzugt sind die UV-Filter Phenylbenzimidazol-5-sulfonsäuresalzen, Ethylhexylsalicylat, Dioctylbutylamidotriazon (INCI: Diethylhexyl-Butamidotriazone), 4,4',4"-(1,3,5-Triazin-2,4,6-triyltriimino)-tris-benzoësäure-tris(2-ethylhexylester) (INCI: Ethylhexyl Triazone).

Die erfindungsgemäß bevorzugten Einsatzkonzentrationen für Phenylbenzimidazol-5-sulfonsäuresalze beträgt von 0,5 bis 4 Gew.-%, bezogen auf das Gesamtgewicht der Zubereitung, die bevorzugte Einsatzkonzentration für Ethylhexylsalicylat beträgt von 0,5 bis 5 Gew.-%, bezogen auf das Gesamtgewicht.

Es ist vorteilhaft im Sinne der vorliegenden Erfindung, wenn die Zubereitung einen oder mehrere UV-Filter a) in einer Gesamtkonzentration von 1 bis 30 Gewichts-%, bezogen auf das Gesamtgewicht der Zubereitung, enthält.

Die erfindungsgemäße Zubereitung kann erfindungsgemäß vorteilhaft in unterschiedlichen Produktformen vorliegen.

In einer ersten vorteilhaften Ausführungsform liegt die Zubereitung in Form eines Sprays vor.

In diesem Fall ist die erfindungsgemäß vorteilhafte Ausführungsform dadurch gekennzeichnet, dass die Sprayformulierung

Carnaubawachs (INCI Copernicia Cerifera Cera) in einer Konzentration von 0,1 bis 4 Gew.-% und hydriertes Rapsöl (INCI: hydrogenated rapeseed oil) in einer Konzentration von 0,1 bis 4 Gew.-%, jeweils bezogen auf das Gesamtgewicht der Zubereitung, enthält.

Liegt die erfindungsgemäße Zubereitung in Form eines Sprays vor, hat diese, erfindungsgemäß bevorzugt eine Viskosität von 50 bis 3000 mPas, bemessen mit (Rheomat R123 von proRheo + Rotor 1 (300-10.000mPas) Rotor 3 (30-1000mPas) 25°C ±0,2°C, Drehzahl (n=62,5min⁻¹ )(gemessen in 150 mL Weithals Schraubdeckelglas)

In einer zweiten vorteilhaften Ausführungsform kann die Zubereitung in Form einer Emulsion vorliegen. In einem solchen Falle ist es erfindungsgemäß von Vorteil, wenn die Emulsion Carnaubawachs (INCI Copernicia Cerifera Cera) in einer Konzentration von 0,1 bis 4 Gew.-% und hydriertes Rapsöl (INCI: hydrogenated rapeseed oil) in einer Konzentration von 0,1 bis 4 Gew.-%, jeweils bezogen auf das Gesamtgewicht der Zubereitung, enthält.

Der erfindungsgemäß vorteilhafte Emulsionstyp ist dabei die O/W-Emulsion und W/O Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate, Natriumstearylglutamat, enthält.

Liegt die erfindungsgemäße Zubereitung in Form einer W/O -Emulsion vor, so ist sie erfindungsgemäß vorteilhaft dadurch gekennzeichnet, dass die Zubereitung einen oder mehrere W/O-Emulgatoren gewählt aus der Gruppe der Verbindungen
Diisostearoyl Polyglyceryl-3 Dimer Dilinoleate, Polyglyceryl-4
Diisostearate/Polyhydroxystearate/Sebacate, Polyglyceryl-3 Polyricinoleate und/oder Polyglyceryl-2 Sesquioleate enthält.

In einer dritten vorteilhaften Ausführungsform kann die Zubereitung in Form einer alkoholischen Zubereitung vorliegen. Die erfindungsgemäß bevorzugte Ausführungsform ist dabei das alkoholische Spray.

Die alkoholische Zubereitung enthält erfindungsgemäß vorteilhaft
Carnaubawachs (INCI Copernicia Cerifera Cera) in einer Konzentration von 0,1 bis 3 Gew.-% und hydriertes Rapsöl (INCI: hydrogenated rapeseed oil) in einer Konzentration von 0,1 bis 3 Gew.-%, jeweils bezogen auf das Gesamtgewicht der Zubereitung.

Als "ethanolische Zubereitung" wird im Rahmen der vorliegenden Erfindung ein Sonnenschutzmittel verstanden, dass mindestens 10 Gewichts-% Ethanol, bezogen auf das Gesamtgewicht der Zubereitung, enthält. Erfindungsgemäß bevorzugt enthält ein solches ethanolisches Sonnenschutzmittel von 20 bis 75 Gewichts-% Ethanol und besonders bevorzugt von 25 bis 70 Gewichts-% Ethanol, jeweils bezogen auf das Gesamtgewicht der Zubereitung.

Erfindungsgemäß bevorzugte Ausführungsformen der vorliegenden Erfindung sind dadurch gekennzeichnet, dass die Zubereitung Cetylpalmitat (INCI: Cetyl Palmitate) in einer Konzentration von 0,1 bis 4 Gew.-%, bezogen auf das Gesamtgewicht der Zubereitung, enthält.

Es ist ferner erfindungsgemäß bevorzugt, wenn die Zubereitung C18-38 Alkylhydroxystearoylstearat (INCI: C18-38 Alkyl Hydroxystearoyl Stearate) in einer Konzentration von 0,1 bis 4 Gew.-%, bezogen auf das Gesamtgewicht der Zubereitung, enthält.

Es ist erfindungsgemäß vorteilhaft, wenn die Zubereitung Xanthangummi (INCI: Xanthan Gum) enthält.

In einem solchen Fall sind die erfindungsgemäß bevorzugten Ausführungsformen dadurch gekennzeichnet, dass die Zubereitung Xanthangummi (INCI: Xanthan Gum) in einer Konzentration von 0,05 bis 2 Gew.-%, bezogen auf das Gesamtgewicht der Zubereitung enthält.

Vorteilhafte Ausführungsformen im Sinne der vorliegenden Erfindung sind dadurch gekennzeichnet, dass die Zubereitung Phenoxyethanol, Ethylhexylglycerin und/oder 4-Hydroxyacetophenon enthält.

Ferner ist es erfindungsgemäß von Vorteil, wenn die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Außerdem ist es erfindungsgemäß vorteilhaft, wenn die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Octocrylen, Parabenen (insbesondere Propyl- und Butylparaben), Methylisothiazolinon, Chlormethyl-isothiazolinon und DMDM-Hydantoin, Polyethylenglycolethern oder Polyethylenglycolestern.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; Thiamidol; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel^{®}1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum und/oder Polyethylen, Nylon.

Es ist erfindungsgemäß bevorzugt, wenn die erfindungsgemäße Zubereitung Tapiocastärke und/oder Distärkephosphat enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat, Butylene Glycol Dicaprylat/Dicaprat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Hydroxypropylmethylcellulose.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Darüber hinaus können die erfindungsgemäßen Zubereitungen die für kosmetische Sonnenschutzmittel üblichen Inhaltsstoffe in den üblichen Einsatzkonzentrationen enthalten.

### Vergleichsversuch

Der erfindungsgemäße Effekt konnte mit dem folgenden Vergleichsversuch belegt werden. Es wurden die folgenden Rezepturen hergestellt.

| inci | **A** | **B** | **C** |
|---|---|---|---|
| Sodium Stearoyl Glutamate + Sodium Chloride | 0,4 | 0,4 | 0,4 |
| Glyceryl Stearate | 1 | 1 | 1 |
| Dibutyl Adipate | 3 | 3 | 3 |
| Isopropyl Palmitate | 6 | 6 | 6 |
| Butylene Glycol Dicaprylate/Dicaprate | 2 | 2 | 2 |
| Cetyl Palmitate | 0,4 | 0,4 | 0,4 |
| Hydrogenated Rapeseed Oil | 0,5 | 0,5 | 0,5 |
| C18-38 Alkyl Hydroxystearoyl Stearate | 0,5 | 0 | 0,5 |
| Copernicia Cerifera Cera | 0 | 0,5 | 0,5 |
| Konservierung (Alcohol Denat. + Aqua, Phenoxyethanol) | 7,4 | 7,4 | 7,4 |
| Hydroxyacetophenone | 0,2 | 0,2 | 0 |
| Xanthan Gum | 0,2 | 0,2 | 0,2 |
| Microcrystalline Cellulose + Cellulose Gum | 1 | 1 | 1 |
| Tocopheryl Acetate | 0,1 | 0,1 | 0,1 |
| UV Filter (Butyl Methoxydibenzoylmethane, Bis-Ethylhexyloxyphenol Methoxyphenyl Triazine, Ethylhexyl Salicylate, Ethylhexyl Triazone, Phenylbenzimidazole Sulfonic Acid) | 11,3 | 11,3 | 11,3 |
| Glycerin | 5 | 5 | 5 |
| Caprylyl Glvcol | 0 | 0 | 0,15 |
| Parfum | 0,5 | 0,5 | 0,5 |
| Aqua + Trisodium EDTA | 1 | 1 | 1 |
| Aqua + Sodium Hydroxide | 0,165 | 0,165 | 0,165 |
| Aqua | ad 100 | ad 100 | ad 100 |
| | | | |
| SPF | 31,5 | **40,5** | **36** |
| SPW [%] | 48 | **54** | **63** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| inci | A | B | C | D | E | F |
|---|---|---|---|---|---|---|
| Sodium Stearoyl Glutamate + Sodium Chloride | 0,2 | 0,2 | | | | |
| Polyglyceryl-10 Stearate | | | | 0,5 | | |
| Glyceryl Stearate Citrate | | | 0,5 | | | |
| Polyglyceryl-3 Methylglucose Distearate | | 0,2 | 0,2 | | 0,2 | |
| Glyceryl Stearate SE | | | | 0,2 | | |
| Sodium Cetearyl Sulfate | | | | 0,4 | | |
| Polyglyceryl-4 Diisostearate/Polyhydroxystearate/Sebacate | | | | | 1 | |
| Dibutyl Adipate | 1 | 3 | 1 | | 2 | 5 |
| Isopropyl Palmitate | 3 | 2 | | 3 | | 10 |
| Butylene Glvcol Dicaprylate/Dicaprate | 4 | | 2 | 3 | 2 | 5 |
| Octyldodecanol | | | | | | 16,825 |
| C12-15 Alkyl Benzoate | 1 | 3 | 3 | 2 | 2 | 9 |
| Propylheptyl Caprylate | | | | | | 5 |
| Glyceryl Stearate | 0,75 | 0,2 | | 0,5 | | |
| Cetyl Palmitate | 2 | | 1 | | 1 | 0,5 |
| Hydrogenated Rapeseed Oil | 1 | 2 | 3 | 0,25 | 0,75 | 0,5 |
| C18-38 Alkyl Hydroxystearoyl Stearate | 0,25 | | | 0,15 | | |
| Copernicia Cerifera Cera | 0,25 | 1 | 0,5 | 3 | 1,12 | 0,5 |
| Synthetic Beeswax | | | | | 0,2 | |
| Hydrogenated Coco-Glycerides | | | | 0,25 | | |
| Synthetic Wax | | | 0,5 | | | |
| Phenylbenzimidazole Sulfonic Acid | 1 | 0,5 | 1 | | 2 | |
| Ethylhexyl Salicylate | 3 | 1 | 2 | 3,3 | 2 | 4,5 |
| Bis-Ethylhexyloxyphenol Methoxyphenyl Triazine | 2,5 | 3 | 2 | 2,5 | 3,5 | 2 |
| Ethylhexyl Triazone | 2 | | 1 | 2 | 1 | |
| Butyl Methoxydibenzoylmethane | 2,5 | 4,75 | 3 | 2,5 | 3 | 3 |
| Diethylamino Hydroxybenzoyl Hexyl Benzoate | 1 | | 1 | 2 | 1 | 3 |
| Homosalate | | | | | | 0,9 |
| Diethylhexyl Butamido Triazone | 1 | 1 | | 1 | 1 | 0,5 |
| Xanthan Gum | 0,1 | 0,15 | 0,3 | 0,25 | 0,4 | 0,05 |
| Microcrystalline Cellulose + Cellulose Gum | 1,5 | 1,5 | 0,75 | 1 | 0,5 | |
| Alcohol Denat. + Aqua | 6 | 4 | 3 | 5 | 7 | 30 |
| Hydroxyacetophenone | 0,1 | 0,2 | 0,35 | 0,1 | | |
| Caprylyl Glycol | | 0,1 | | | | |
| Phenoxyethanol | 0,2 | | 0,2 | | 0,5 | |
| Aqua + Trisodium EDTA | 0,5 | 0,25 | | 0,3 | | 0,125 |
| Ethylhexylglycerin | | | | 0,2 | | |
| Aqua + Sodium Hydroxide | 0,33 | 0,165 | 0,33 | | 0,66 | |
| Tocopheryl Acetate | 0,5 | 1 | 0,75 | 0,5 | | 2 |
| Glycerin | 3 | 5 | 7 | 3 | 4 | 1 |
| Parfum | 0,4 | 0,2 | 0,5 | 0,3 | 0,5 | 0,6 |
| Aqua | ad 100 | ad 100 | ad 100 | ad 100 | ad 100 | |

## Patentansprüche

1. Kosmetisches Sonnenschutzmittel enthaltend
a) ein oder mehrere UV-Filter,
b) Carnaubawachs (INCI Copernicia Cerifera Cera)
c) hydriertes Rapsöl (INCI: hydrogenated rapeseed oil),
**dadurch gekennzeichnet, dass** die Zubereitung bzw. das Sonnenschutzmittel zusätzlich Cetylpalmitat (INCI: Cetyl Palmitate) und/oder C18-38 Alkylhydroxystearoylstearat (INCI: C18-38 Alkyl Hydroxystearoyl Stearate) enthält.

2. Kosmetisches Sonnenschutzmittel enthaltend
a) ein oder mehrere UV-Filter,
b) Carnaubawachs (INCI Copernicia Cerifera Cera)
c) hydriertes Rapsöl (INCI: hydrogenated rapeseed oil), **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

3. Verwendung einer Kombination aus Sonnenschutzmittel Carnaubawachs (INCI Copernicia Cerifera Cera) und hydriertes Rapsöl (INCI: hydrogenated rapeseed oil) zur Erhöhung der Wasserfestigkeit kosmetischer Sonnenschutzmittel.

4. Zubereitung oder Verwendung nach einem der vorhergehenden Ansprüche, **dadurch gekennzeichnet, dass** die Zubereitung bzw. das Sonnenschutzmittel frei ist von Polyacrylaten, Carbomeren und Polyvinylpyrrolidonen.

5. Verwendung nach einem der vorhergehenden Ansprüche, **dadurch gekennzeichnet, dass** die Zubereitung bzw. das Sonnenschutzmittel zusätzlich Cetylpalmitat (INCI: Cetyl Palmitate) und/oder C18-38 Alkylhydroxystearoylstearat (INCI: C18-38 Alkyl Hydroxystearoyl Stearate) enthält.

6. Kosmetische Zubereitung oder Verwendung nach einem der vorhergehenden Ansprüche, **dadurch gekennzeichnet, dass** die Zubereitung einen oder mehrere UV-Filter gewählt aus der Gruppe der Verbindungen 4-(tert.-Butyl)-4'-methoxydiben-zoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

7. Kosmetische Zubereitung oder Verwendung nach einem der vorhergehenden Ansprüche, **dadurch gekennzeichnet, dass** sie einen oder mehrere UV-Filter enthält, gewählt aus der Gruppe der Verbindungen 2-Phenylbenzimidazol-5-sulfonsäuresalze; Phenylen-1,4-bis-(2-benzimidazyl)-3,3'-5,5'-tetrasulfonsäuresalze;
1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; Ethylhexylsalicylat; Terephthalidendicamphersulfonsäuresalze; 2-Ethylhexyl-2-cyano-3,3-diphenylacrylat; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid.

8. Kosmetische Zubereitung oder Verwendung nach einem der vorhergehenden Ansprüche, **dadurch gekennzeichnet, dass** die Zubereitung einen oder mehrere UV-Filter a) in einer Gesamtkonzentration von 1 bis 30 Gewichts-%, bezogen auf das Gesamtgewicht der Zubereitung, enthält.

9. Kosmetische Zubereitung oder Verwendung nach einem der vorhergehenden Ansprüche, **dadurch gekennzeichnet, dass** die Zubereitung in Form eines Sprays vorliegt.

10. Kosmetische Zubereitung oder Verwendung nach Anspruch 9, **dadurch gekennzeichnet, dass** Carnaubawachs (INCI Copernicia Cerifera Cera) in einer Konzentration von 0,1 bis 4,0 Gew.-% und hydriertes Rapsöl (INCI: hydrogenated rapeseed oil) in einer Konzentration von 0,1 bis 4,0 Gew.-%, jeweils bezogen auf das Gesamtgewicht der Zubereitung, enthält.

11. Kosmetische Zubereitung oder Verwendung nach einem der vorhergehenden Ansprüche 1 bis 8, **dadurch gekennzeichnet, dass** die Zubereitung in Form einer Emulsion oder einer alkoholischen Zubereitung vorliegt.

12. Kosmetische Zubereitung oder Verwendung nach Anspruch 11, wobei die Zubereitung in Form einer Emulsion vorliegt, **dadurch gekennzeichnet, dass** Carnaubawachs (INCI Copernicia Cerifera Cera) in einer Konzentration von 0,1 bis 4,0 Gew.-% und hydriertes Rapsöl (INCI: hydrogenated rapeseed oil) in einer Konzentration von 0,1 bis 4,0 Gew.-%, jeweils bezogen auf das Gesamtgewicht der Zubereitung, enthält.

13. Kosmetische Zubereitung oder Verwendung nach Anspruch 11, wobei die Zubereitung in Form einer alkoholischen Zubereitung vorliegt, **dadurch gekennzeichnet, dass** Carnaubawachs (INCI Copernicia Cerifera Cera) in einer Konzentration von 0,1 bis 3,0 Gew.-% und hydriertes Rapsöl (INCI: hydrogenated rapeseed oil) in einer Konzentration von 0,1 bis 3,0 Gew.-%, jeweils bezogen auf das Gesamtgewicht der Zubereitung, enthält.

14. Kosmetische Zubereitung oder Verwendung nach einem der vorhergehenden Ansprüche, **dadurch gekennzeichnet, dass** die Zubereitung Cetylpalmitat (INCI: Cetyl Palmitate) in einer Konzentration von 0,1 bis 4,0 Gew.-%, bezogen auf das Gesamtgewicht der Zubereitung, enthält

15. Kosmetische Zubereitung oder Verwendung nach einem der vorhergehenden Ansprüche, **dadurch gekennzeichnet, dass** die Zubereitung C18-38 Alkylhydroxystearoylstearat (INCI: C18-38 Alkyl Hydroxystearoyl Stearate) in einer Konzentration von 0,1 bis 4,0 Gew.-%, bezogen auf das Gesamtgewicht der Zubereitung, enthält.

16. Kosmetische Zubereitung oder Verwendung nach einem der vorhergehenden Ansprüche, **dadurch gekennzeichnet, dass** die Zubereitung Xanthangummi (INCI: Xanthan Gum) enthält.

17. Kosmetische Zubereitung oder Verwendung nach einem der vorhergehenden Ansprüche, **dadurch gekennzeichnet, dass** die Zubereitung Xanthangummi (INCI: Xanthan Gum) in einer Konzentration von 0,05 bis 2,0 Gew.-%, bezogen auf das Gesamtgewicht der Zubereitung enthält.

18. Kosmetische Zubereitung oder Verwendung nach einem der vorhergehenden Ansprüche, **dadurch gekennzeichnet, dass** die Zubereitung Phenoxyethanol, Ethylhexylglycerin und/oder 4-Hydroxyacetophenon enthält.

19. Verwendung nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

20. Kosmetische Zubereitung oder Verwendung nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Octocrylen, Parabenen, Methylisothiazolinon, Chlormethyl-isothiazolinon und DMDM-Hydantoin, Polyethylenglycolethern oder Polyethylenglycolestern.

## Claims

1. Cosmetic sunscreen comprising
a) one or more UV filters,
b) carnauba wax (INCI: Copernicia Cerifera Cera),
c) hydrogenated rapeseed oil (INCI: Hydrogenated Rapeseed Oil),
**characterized in that** the preparation or the sunscreen additionally comprises cetyl palmitate (INCI: Cetyl Palmitate) and/or C18-38 alkyl hydroxystearoyl stearate (INCI: C18-38 Alkyl Hydroxystearoyl Stearate).

2. Cosmetic sunscreen comprising
a) one or more UV filters,
b) carnauba wax (INCI: Copernicia Cerifera Cera),
c) hydrogenated rapeseed oil (INCI: Hydrogenated Rapeseed Oil), **characterized in that** the preparation comprises one or more alkanediols from the following group of compounds: pentane-1,2-diol, hexane-1,2-diol, octane-1,2-diol, decane-1,2-diol, 2-methylpropane-1,3-diol.

3. Use of a combination of sunscreen camauba wax (INCI: Copernicia Cerifera Cera) and hydrogenated rapeseed oil (INCI: Hydrogenated Rapeseed Oil) to increase the water resistance of cosmetic sunscreens.

4. Preparation or use according to any of the preceding claims, **characterized in that** the preparation or the sunscreen is free of polyacrylates, carbomers and polyvinylpyrrolidones.

5. Use according to any of the preceding claims, **characterized in that** the preparation or the sunscreen additionally comprises cetyl palmitate (INCI: Cetyl Palmitate) and/or C18-38 alkyl hydroxystearoyl stearate (INCI: C18-38 Alkyl Hydroxystearoyl Stearate).

6. Cosmetic preparation or use according to any of the preceding claims, **characterized in that** the preparation comprises one or more UV filters selected from the following group of compounds: 4-(tert-butyl)-4'-methoxydibenzoylmethane (INCI: Butyl Methoxydibenzoylmethane), hexyl 2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

7. Cosmetic preparation or use according to any of the preceding claims, **characterized in that** it comprises one or more UV filters selected from the following group of compounds: 2-phenylbenzimidazole-5-sulfonic acid salts; phenylene-1,4-bis(2-benzimidazyl)-3,3'-5,5'-tetrasulfonic acid salts; 1,4-di(2-oxo-10-sulfo-3-bomylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; ethylhexyl salicylate; terephthalidenedicamphorsulfonic acid salts; 2-ethylhexyl 2-cyano-3,3-diphenylacrylate; amyl 4-(dimethylamino)benzoate; di(2-ethylhexyl) 4-methoxybenzalmalonate; isoamyl 4-methoxycinnamate; 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; dioctyl butylamido triazone (INCI: Diethylhexyl Butamido Triazone); 2,4-bis[5-1(dimethylpropyl)benzoxazol-2-yl-(4-phenyl)imino]-6-(2-ethylhexyl)imino-1,3,5-triazine (with CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tris(biphenyl-4-yl)-1,3,5-triazine; merocyanines; piperazine derivatives; titanium dioxide; zinc oxide.

8. Cosmetic preparation or use according to any of the preceding claims, **characterized in that** the preparation comprises one or more UV filters a) in a total concentration of 1% to 30% by weight, based on the total weight of the preparation.

9. Cosmetic preparation or use according to any of the preceding claims, **characterized in that** the preparation is in the form of a spray.

10. Cosmetic preparation or use according to Claim 9, **characterized in that** comprises carnauba wax (INCI: Copernicia Cerifera Cera) in a concentration of 0.1% to 4.0% by weight and hydrogenated rapeseed oil (INCI: Hydrogenated Rapeseed Oil) in a concentration of 0.1% to 4.0% by weight, in each case based on the total weight of the preparation.

11. Cosmetic preparation or use according to any of the preceding Claims 1 to 8, **characterized in that** the preparation is in the form of an emulsion or an alcoholic preparation.

12. Cosmetic preparation or use according to Claim 11, wherein the preparation is in the form of an emulsion, **characterized in that** comprises carnauba wax (INCI: Copernicia Cerifera Cera) in a concentration of 0.1% to 4.0% by weight and hydrogenated rapeseed oil (INCI: Hydrogenated Rapeseed Oil) in a concentration of 0.1% to 4.0% by weight, in each case based on the total weight of the preparation.

13. Cosmetic preparation or use according to Claim 11, wherein the preparation is in the form of an alcoholic preparation, **characterized in that** comprises carnauba wax (INCI: Copernicia Cerifera Cera) in a concentration of 0.1% to 3.0% by weight and hydrogenated rapeseed oil (INCI: Hydrogenated Rapeseed Oil) in a concentration of 0.1% to 3.0% by weight, in each case based on the total weight of the preparation.

14. Cosmetic preparation or use according to any of the preceding claims, **characterized in that** the preparation comprises cetyl palmitate (INCI: Cetyl Palmitate) in a concentration of 0.1% to 4.0% by weight, based on the total weight of the preparation.

15. Cosmetic preparation or use according to any of the preceding claims, **characterized in that** the preparation comprises C18-38 alkyl hydroxystearoyl stearate (INCI: C18-38 Alkyl Hydroxystearoyl Stearate) in a concentration of 0.1% to 4.0% by weight, based on the total weight of the preparation.

16. Cosmetic preparation or use according to any of the preceding claims, **characterized in that** the preparation comprises xanthan gum (INCI: Xanthan Gum).

17. Cosmetic preparation or use according to any of the preceding claims, **characterized in that** the preparation comprises xanthan gum (INCI: Xanthan Gum) in a concentration of 0.05% to 2.0% by weight, based on the total weight of the preparation.

18. Cosmetic preparation or use according to any of the preceding claims, **characterized in that** the preparation comprises phenoxyethanol, ethylhexylglycerin and/or 4-hydroxyacetophenone.

19. Use according to any of the preceding claims, **characterized in that** the preparation comprises one or more alkanediols from the following group of compounds: pentane-1,2-diol, hexane-1,2-diol, octane-1,2-diol, decane-1,2-diol, 2-methylpropane-1,3-diol.

20. Cosmetic preparation or use according to any of the preceding claims, **characterized in that** the preparation is free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), octocrylene, parabens, methylisothiazolinone, chloromethylisothiazolinone and DMDM hydantoin, polyethylene glycol ethers or polyethylene glycol esters.

## Revendications

1. Agent cosmétique de protection solaire, contenant
a) un ou plusieurs filtres UV,
b) de la cire de carnauba (INCI : Copernica Cerifera Cera)
c) de l'huile de colza hydrogénée (INCI : hydrogenated rapeseed oil),
**caractérisé en ce que** la préparation ou, selon le cas, l'agent de protection solaire contient en plus du palmitate de cétyle, (INCI : Cetyl Palmitate) et/ou de l'hydroxystéaroylstéarate de C18-38-alkyle (INCI : C18-38 Alkyl Hydroxystearoyl Stearate).

2. Agent cosmétique de protection solaire, contenant
a) un ou plusieurs filtres UV,
b) de la cire de carnauba (INCI : Copernica Cerifera Cera)
c) de l'huile de colza hydrogénée (INCI : hydrogenated rapeseed oil), **caractérisé en ce que** la préparation contient un ou plusieurs alcanediols du groupe des composés 1,2-pentanediol, 1,2-hexanediol, 1,2-octanediol, 1,2-décanediol, 2-méthyl-1,3-propanediol,

3. Utilisation d'une combinaison d'agent de protection solaire cire de carnauba (INCI : Copernicia Cerifera Cera) et d'huile de colza hydrogénée (INCI : hydrogenated rapeseed oil) pour l'augmentation de la résistance à l'eau d'agents cosmétiques de protection solaire.

4. Préparation ou utilisation selon l'une des revendications précédentes, **caractérisée en ce que** la préparation ou, selon le cas, l'agent de protection solaire est exempt de polyacrylates, de carbomères et de polyvinylpyrrolidones.

5. Utilisation selon l'une des revendications précédentes, **caractérisée en ce que** la préparation ou, selon le cas, l'agent de protection solaire contient en plus du palmitate de cétyle, (INCI : Cetyl Palmitate) et/ou du hydroxystéaroylstéarate de C18-38-alkyle (INCI : C18-38 Alkyl Hydroxystearoyl Stearate).

6. Préparation cosmétique ou utilisation selon l'une des revendications précédentes, **caractérisée en ce que** la préparation contient un ou plusieurs filtres UV, choisis dans le groupe des composés 4-(tert-butyl)-4'-méthoxydibenzoylméthane (INCI : Butyl Methoxydibenzoylmethane), ester hexylique de l'acide 2-[-4-(diéthylamino)-2-hydroxybenzoyl]benzoïque (INCI : Diethylamino Hydroxybenzoyl Hexyl Benzoate) et 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

7. Préparation cosmétique ou utilisation selon l'une des revendications précédentes, **caractérisée en ce qu'**elle contient un ou plusieurs filtres UV supplémentaires, choisis dans le groupe constitué par les composés sels de l'acide 2-phénylbenzimidazole-5-sulfonique ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidène-méthyl)benzène-sulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; salicylate d'éthylhexyle ; sels de l'acide téréphtalidène-dicamphre-sulfonique ; 2-cyano-3,3-diphénylacrylate de 2-éthylhexyle ; ester amylique de l'acide 4-(diméthylamino)benzoïque ; ester di(2-éthylhexylique) de l'acide 4-méthoxybenzalmalonique ; ester isoamylique de l'acide 4-méthoxycinnamique ; 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (n° CAS 288254-16-0) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine, mérocyanine ; dérivés de pipérazine ; dioxyde de titane ; oxyde de zinc.

8. Préparation cosmétique ou utilisation selon l'une des revendications précédentes, **caractérisée en ce que** la préparation contient un ou plusieurs filtres UV a) en une concentration totale de 1 à 30% en poids par rapport au poids total de la préparation.

9. Préparation cosmétique ou utilisation selon l'une des revendications précédentes, **caractérisée en ce que** la préparation cosmétique se trouve sous forme d'un spray.

10. Préparation cosmétique ou utilisation selon la revendication 9, **caractérisée en ce que** contient la cire de carnauba (INCI : Copernicia Cerifera Cera) en une concentration de 0,1 à 4,0% en poids et l'huile de colza hydrogénée (INCI : hydrogenated rapeseed oil) en une concentration de 0,1 à 4,0% en poids, à chaque fois par rapport au poids total de la préparation.

11. Préparation cosmétique ou utilisation selon l'une des revendications précédentes 1 à 8, **caractérisée en ce que** la préparation cosmétique se trouve sous forme d'une émulsion ou d'une préparation alcoolique.

12. Préparation cosmétique ou utilisation selon la revendication 11, la préparation se trouvant sous forme d'une émulsion, **caractérisée en ce que** contient la cire de carnauba (INCI : Copernicia Cerifera Cera) en une concentration de 0,1 à 4,0% en poids et l'huile de colza hydrogénée (INCI : hydrogenated rapeseed oil) en une concentration de 0,1 à 4,0% en poids, à chaque fois par rapport au poids total de la préparation.

13. Préparation cosmétique ou utilisation selon la revendication 11, la préparation se trouvant sous forme d'une préparation alcoolique, **caractérisée en ce que** contient la cire de camauba (INCI : Copemicia Cerifera Cera) en une concentration de 0,1 à 3,0% en poids et l'huile de colza hydrogénée (INCI : hydrogenated rapeseed oil) en une concentration de 0,1 à 3,0% en poids, à chaque fois par rapport au poids total de la préparation.

14. Préparation cosmétique ou utilisation selon l'une des revendications précédentes, **caractérisée en ce que** la préparation contient du palmitate de cétyle (INCI : Cetyl Palmitate) en une concentration de 0,1 à 4,0% en poids, par rapport au poids total de la préparation.

15. Préparation cosmétique ou utilisation selon l'une des revendications précédentes, **caractérisée en ce que** la préparation contient de l'hydroxystéaroylstéarate de C18-38-alkyle (INCI : C18-38 Alkyl Hydroxystearoyl Stearate) en une concentration de 0,1 à 4,0% en poids par rapport au poids total de la préparation.

16. Préparation cosmétique ou utilisation selon l'une des revendications précédentes, **caractérisée en ce que** la préparation contient de la gomme de xanthane (INCI : Xanthan Gum).

17. Préparation cosmétique ou utilisation selon l'une des revendications précédentes, **caractérisée en ce que** la préparation contient de la gomme de xanthane (INCI : Xanthan Gum) en une concentration de 0,05 à 2,0% en poids, par rapport au poids total de la préparation.

18. Préparation cosmétique ou utilisation selon l'une des revendications précédentes, **caractérisée en ce que** la préparation contient du phénoxyéthanol, de l'éthylhexylglycérol et/ou de la 4-hydroxyacétophénone.

19. Utilisation selon l'une des revendications précédentes, **caractérisée en ce que** la préparation contient un ou plusieurs alcanediols du groupe des composés 1,2-pentanediol, 1,2-hexanediol, 1,2-octanediol, 1,2-décanediol, 2-méthyl-1,3-propanediol.

20. Préparation cosmétique ou utilisation selon l'une des revendications précédentes, **caractérisée en ce que** la préparation est exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), d'octocrylène, de parabènes, de méthylisothiazolinone, de chlorométhylisothiazolinone et de DMDM-hydantoïne, de polyéthylèneglycoléthers ou d'esters de polyéthylèneglycol.
